# EUROPEAN PATENT APPLICATION

(11) **EP 3 090 689 A1**
(43) Date of publication of application: **09.11.2016**
(21) Application number: 14877006.8
(22) Date of filing: 19.12.2014
(51) Int. Cl.: A61B 17/072

(54) **LINEAR SUTURING AND CUTTING DEVICE**

(30) Priority: 31.12.2013 CN 201320885569 U; 31.12.2013 CN 201310747332
(71) Applicant: Suzhou Touchstone International Medical Science Co., Ltd., Suzhou, Jiangsu 215021 (CN)
(72) Inventor: CHEN, Wangdong, Suzhou Jiangsu 215021 (CN); SHAN, Teng, Suzhou Jiangsu 215021 (CN)
(74) Representative: Kordel, Mattias
(86) International application number: PCT/CN2014/094365
(87) International publication number: WO 2015/101184

(57) **Abstract**

A linear suturing and cutting device (100), comprising an upper forceps holder (101) and a lower forceps holder (102) that can be closed and opened mutually. The upper forceps holder (101) comprises a nail anvil (10). The lower forceps holder (102) comprises a nail cartridge rack (103), and the nail cartridge rack (103) is detachably provided with a nail cartridge (20). The inside of the nail cartridge (20) is provided with a nail cartridge hole (21) which runs through the surface of the nail cartridge (20) and a nail pushing sheet (40) which is arranged inside the nail cartridge hole (21) and can slide relative to the nail cartridge hole (21). The inside of the nail cartridge rack (103) is further slidably provided with a trigger sheet (30), and the trigger sheet (30) is provided with a wedge-shaped nail pushing unit (31) which can drive the nail pushing sheet (40) to slide inside the nail cartridge hole (21). The nail cartridge hole (21) is inclined towards the far end of the nail cartridge (20). In this way, when the nail pushing sheet (40) inside the nail cartridge hole (21) is pushed, the stress direction of the nail pushing sheet (40) is identical to the extension direction of the nail cartridge hole (21), and relatively large friction force between the nail pushing sheet (40) and the inner wall of the nail cartridge hole (21) can be avoided, so that the nail pushing sheet (40) can be pushed more smoothly, and the surgical difficulty can be reduced.

## Description

### Technical Field

The invention belongs to the technical field of medical apparatus and instruments and particularly relates to a linear suturing and cutting device.

### Background

In the prior art, in order to obtain a good coordination between the firing piece and the staple pushers, the end portion of the firing piece is usually configured to be in wedge shape for well guiding the pushing movements of staple pushers, which consequently brings some problems: since the cartridge holes are arranged vertically, the wedge shape portion of the firing piece will apply an inclined driving force to the staple pushers during its coordination with the staple pushers. So, when the staple pushers are moving in the cartridge holes, the interaction force between the side wall of the cartridge holes and the staple pushers is relatively strong, which increases the difficulty of pushing the staples out.

### Summary

An object of the invention is provided a linear suturing and cutting device with the staples being pushed out of the cartridge holes more easily.

For realizing the above-mentioned object, the invention provides a linear suturing and cutting device, comprising an upper jaw and a lower jaw capable of being closed or opened relative to each other; said upper jaw includes an anvil; said lower jaw includes a staple cartridge frame, said staple cartridge frame is detachably provided with a staple cartridge, said staple cartridge is arranged with cartridge holes inside which run throughout said staple cartridge to its working surface and staple pushers correspondingly disposed inside said cartridge holes and being capable of sliding relative to said cartridge holes, said staple cartridge frame is slidably provided with a firing piece inside, said firing piece is configured with a wedge-shape staple pushing member capable of driving said staple pushers to slide in said cartridge holes, wherein, said cartridge holes are configured to be inclined towards distal end of said staple cartridge.

As a further aspect, said firing piece moves from the proximal end to the distal end of said staple cartridge, said wedge-shape staple pushing member has a staple pushing surface engaged with said staple pushers, said staple pushing surface is configured to be sloped upward in the direction from the distal end towards the proximal end.

As a further aspect, said cartridge holes are vertically arranged relative to said staple pushing surface.

As a further aspect, upper end surface of each said cartridge hole is an upward inclined plane in the extending direction from the distal end towards the proximal end of the staple cartridge.

As a further aspect, said anvil includes a first anvil unit and at least one second anvil unit which is fixed at either side of said first anvil unit, and said first anvil unit and said second anvil unit are manufactured separately.

As a further aspect, said staple cartridge includes a cutter groove arranged along the major longitudinal axis thereof, the pattern of said cartridge holes by either side of said cutter groove presents as that each two adjacent rows of cartridge holes are in a staggered arrangement.

As a further aspect, the patterns of said cartridge holes by two side of said cutter groove are symmetrical with each other.

As a further aspect, said anvil has a staple forming surface disposed opposite to the staple cartridge, wherein, part of said staple forming surface corresponding to said first anvil unit and part of said staple forming surface corresponding to said second anvil unit are in wave shape.

As a further aspect, said staple forming surface is configured for engaging with said working surface of said staple cartridge.

The above-mentioned object of the invention can also be realized by a method below, providing a linear suturing and cutting device, comprising an upper jaw and a lower jaw capable of being closed or opened relative to each other, said upper jaw includes an anvil, said lower jaw includes a staple cartridge frame, said staple cartridge frame is detachably provided with a staple cartridge, said staple cartridge is arranged with cartridge holes inside which run throughout said staple cartridge to its working surface, and staple pushers correspondingly arranged inside said cartridge holes and being capable of sliding relative to said cartridge holes, said staple cartridge frame is slidably provided with a firing piece inside, said firing piece is configured with a wedge-shape staple pushing member capable of driving said staple pushers to slide in said cartridge holes, wherein, said cartridge holes are configured such that the direction of pushing force received by said staple pushers, while said wedge-shape staple pushing member drives said staple pushers to slide in said cartridge holes, is same with the extending direction of said cartridge holes.

As a further aspect, said cartridge holes are configured to be inclined towards the distal end or proximal end of said staple cartridge.

As a further aspect, said wedge-shape staple pushing member has a staple pushing surface engaged with said staple pushers, said cartridge holes are perpendicular to said staple pushing surface.

Compared with the prior art, the linear suturing and cutting device provided by the present invention comprises such structure that the cartridge holes in staple cartridge is configured to be inclined towards the distal end of said staple cartridge, then, the direction of force received by the staple pushers is identical with the extending direction of cartridge holes during the staple pushers being pushed in the cartridge holes, thus the staple pushers will not get too large friction from the inner wall of cartridge holes and then can be pushed more smoothly, which reduces the difficulty of operation.

### Brief description of the drawings

Fig. 1 is a view of a linear suturing and cutting device according to an embodiment of the present invention;
Fig. 2 is an enlarged view of the portion enclosed in imaginary line shown in Fig. 1;
Fig. 3 is a partial cross-sectional view of the staple cartridge according to an embodiment of the present invention;
Fig. 4 is a top view of the staple cartridge according to an embodiment of the present invention;
Fig. 5 is a view of the anvil according to an embodiment of the present invention;
Fig. 6 is an enlarged view of the portion enclosed in imaginary line shown in Fig. 5.

### Detailed description

Hereinafter, embodiments are described in detail with reference to the accompanying drawings. However, these embodiments can't be used for limiting the scope of present invention. Any other equivalent deformations or modifications of structures, methods or functions which are made by the technical persons in the art according to these embodiments are all intended to be included in the scope of invention.

The terms describing places or directions in the description are determined by taking the position of operator as reference, wherein, the "proximal end" is the end close to operator, and the "distal end" is the end far away from operator.

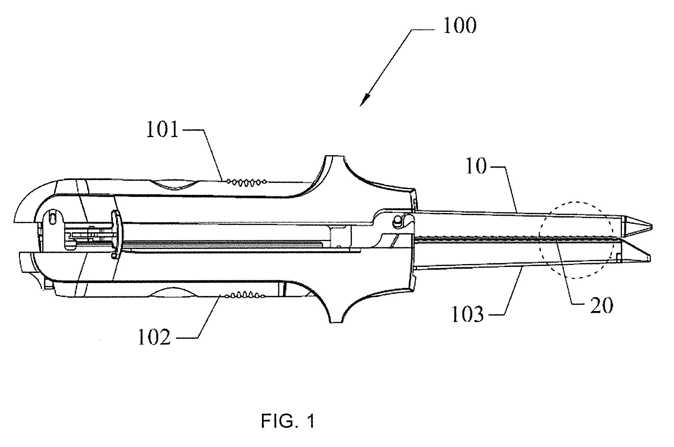

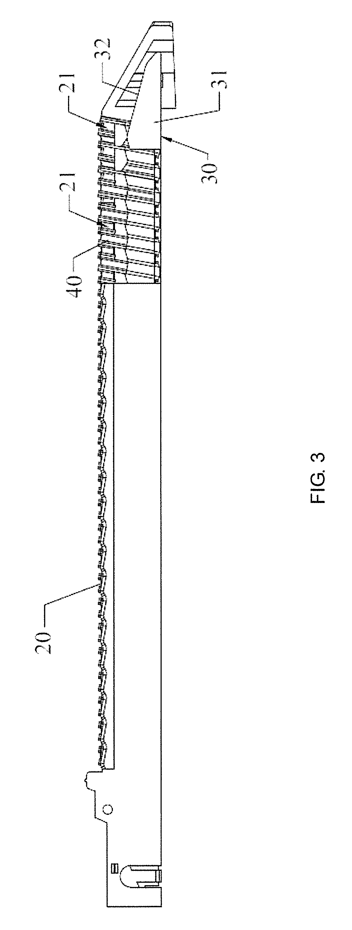

Referring to Fig. 1 to Fig. 3, a linear suturing and cutting device 100 according to one embodiment of the present invention is presented. In this embodiment, the linear suturing and cutting device 100 comprises an upper jaw 101 and a lower jaw 102 capable of being closed or opened relative to each other. It should be noted that, the term "upper" and "lower" herein do not mean to imply to absolutely differentiate the relationship in position, and merely for convenience in description.

The upper jaw 101 includes an anvil 10. The lower jaw 102 includes a staple cartridge frame 103. The staple cartridge frame 103 is detachably provided with a staple cartridge 20. The upper jaw 101 and the lower jaw 102 can be closed or opened in relative to each other to clamp the target tissues between the anvil 10 and the staple cartridge 20. A cutter pushing rod (not shown) and a cutter (not shown) disposed at the distal end of the cutter pushing rod are moveably arranged in the staple cartridge frame 103. The cutter can move from the proximal end to the distal end of the staple cartridge 20 with the force of the cutter pushing rod to cut off the target tissues between the anvil 10 and the staple cartridge 20. And, the staple cartridge 20 is arranged with cartridge holes 21 inside which run throughout the staple cartridge 20 to its working surface and staple pushers 40 disposed in the cartridge holes 21 and being capable of sliding relative to the cartridge holes 21, the staple cartridge frame 103 is slidably provided with a firing piece 30 inside, which is configured with a wedge-shape staple pushing member 31 capable of driving the staple pushers 40 to slide in the cartridge holes 21. When the firing piece 30 moves from the proximal end to the distal end of the staple cartridge 20 with the force of power mechanism, the wedge-shape staple pushing member 31 of the firing piece 30 pushes the staple pushers 40 to move along the cartridge holes 21 so as to fire the staples to suture the tissues.

In this embodiment, the cartridge holes 21 are configured to be inclined towards the distal end of said staple cartridge 20. Since the surface of the wedge-shape staple pushing member 31 engaged with the staple pushers 40 is an inclined staple pushing surface 32 and configured to be sloped upward in the direction from the distal end towards the proximal end, when the wedge-shape staple pushing member 31 moves from the proximal end towards the distal end to push the staple pushers 40 to move, the driving force applied on the staple pushers 40 is also inclined substantially. With the cartridge holes 21 configured to be inclined towards the distal end of the staple cartridge 20, it is possible to reduce the force received by the staple pushers 40 in the direction which is different from the extending direction of the cartridge holes 21, and reduce the friction between the staple pushers 40 and the inner wall of the cartridge holes 21 during the movement of staple pushers 40, to push the staple pushers 40 out more smoothly.

The wedge-shape staple pushing member 31 has a staple pushing surface 32 engaged with the staple pushers 40, which is acted on the staple pushers 40 directly and applies a force to the staple pushers 40 in the direction which is vertical to the staple pushing surface 32. As a preferred embodiment, in this embodiment, the cartridge holes 21 are vertically arranged relative to the staple pushing surface 32, so as to ensure that the direction of pushing force received by the staple pushers 40 is identical with the extending direction of the cartridge holes 21, which may minimize the friction force between the staple pushers 40 and the inner wall of the cartridge holes 21 during the movement of staple pushers 40 to provide the best smoothness for pushing staples.

In the conventional linear suturing and cutting device, the surfaces of the anvil and the staple cartridge which are disposed face to face are designed to be relatively flat. When a doctor performs surgery, the target tissues clamped between the anvil and the staple cartridge may slide or shift to a certain extent. To overcome the above-mentioned disadvantage, in the present embodiment, the upper end surface 211 of each cartridge hole 21 is an upward inclined plane (i.e., the distance to the bottom of the staple cartridge 20 gradually increases) in the extending direction from the distal end towards the proximal end of the staple cartridge 20. In this way, the working surface of the staple cartridge 20 (i.e., the end surface of cartridge hole 21) is configured correspondingly to be uneven, which can get the target tissues to be clamped between the anvil 10 and the staple cartridge 20 more firmly. Besides, such a structure is also in concert with the arrangement of the cartridge holes 21 inclined towards the distal end of the staple cartridge 20 in the present embodiment.

In an alternative embodiment, the upper end surface of the cartridge holes is configured to be in other form of inclined state to achieve the similar technical effects of the present embodiment. For example, the cartridge holes 21 are configured to be inclined towards the proximal end of the staple cartridge 20, and the firing piece 30 moves from the distal end to the proximal end of the staple cartridge 20, and the wedge-shape staple pushing member 31 has a staple pushing surface 32 engaged with the staple pushers, and the staple pushing surface 32 is configured to be sloped downward in the direction from the distal end towards the proximal end. Such a structure can also achieve the technical effects of reducing the friction between the staple pushers and the inner wall of the cartridge holes during the movement of staple pushers 40. But all these variants should be regarded as equivalent variants based on the technical innovation in the present invention, which also should be included within the scope of the present invention.

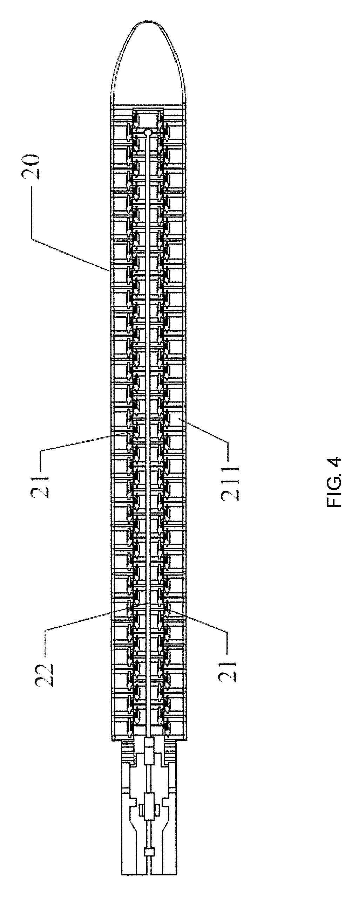

Referring to Fig. 4, the staple cartridge 20 includes a cutter groove 22 arranged along the major longitudinal axis thereof. The pattern of cartridge holes 21 by either side of the cutter groove 22 presents as that each two adjacent rows of cartridge holes 21 are in a staggered arrangement, which is also for the purpose of better clamping effect and suturing effect on the target tissues when the staple cartridge 20 and the anvil 10 are closed.

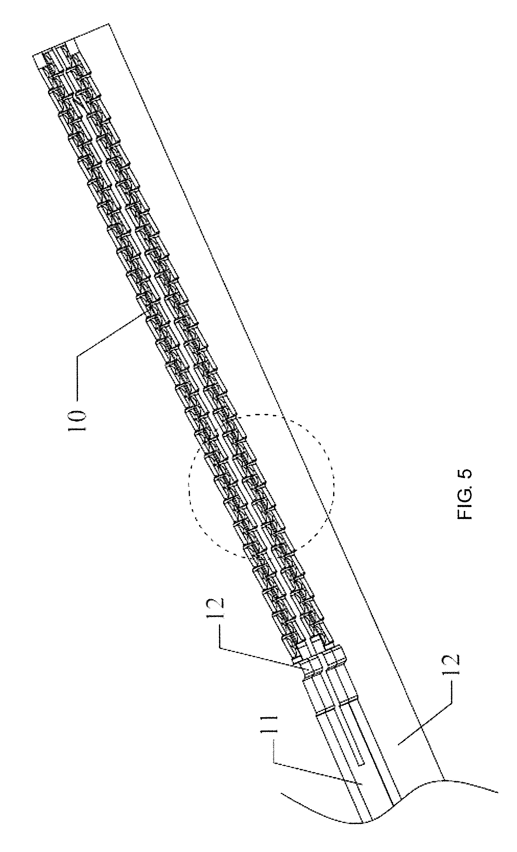

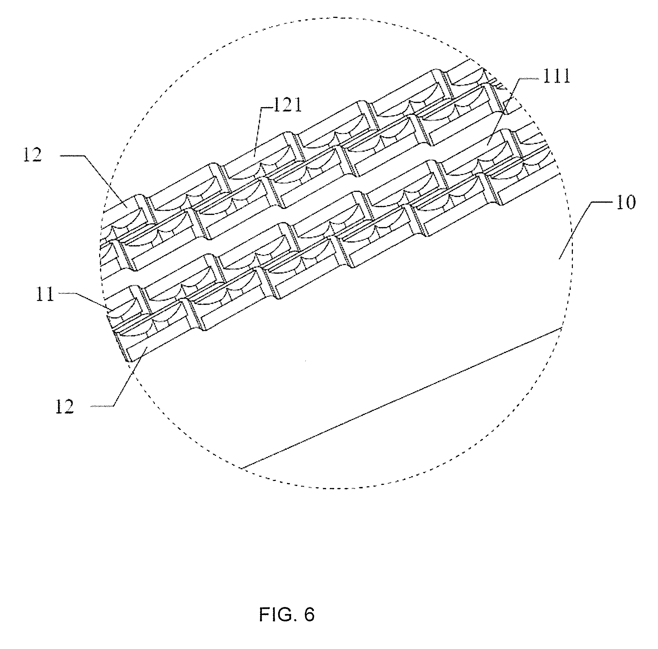

Referring to Fig. 5 and Fig. 6, particularly, in the present embodiment, the anvil 10 includes a first anvil unit 11 and at least one second anvil unit 12 which is fixed at either side of the first anvil unit 11, and the first anvil unit 11 and the second anvil units 12 are manufactured separately. It should be noted that, in the present embodiment, taking the staple cartridge 20 having four rows of the cartridge holes 21 as an example, the anvil 10 is correspondingly arranged with a first anvil unit 11 and two second anvil units 12. In another alternative embodiment, for example, in the linear suturing and cutting device having 6 rows of cartridge holes, it is necessary to set more second anvil units correspondingly.

In the present embodiment, since the pattern of cartridge holes by either side of the cutter groove 22 are in a staggered arrangement, the anvil can be obtained by manufacturing the first anvil unit and the second anvil unit separately for reducing the forming difficulty of the anvil. In the actual manufacture, the first anvil unit 11 and the second anvil unit 12 manufactured separately may be fitted together by subsequent positioning welding, which is convenient for assembly.

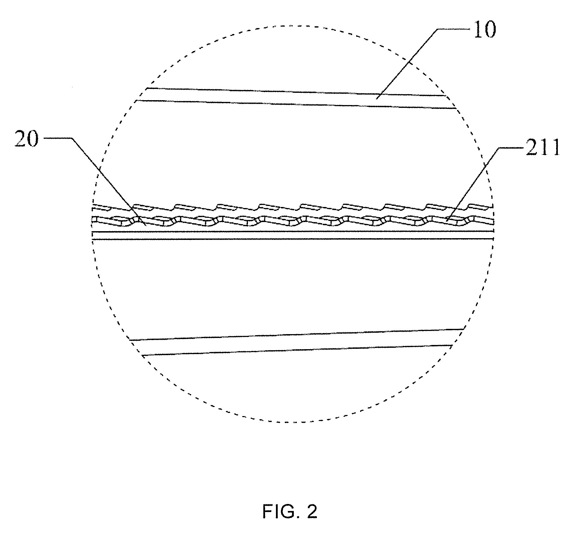

The patterns of cartridge holes 21 by two sides of the cutter groove 22 are symmetrically with each other, such arrangement can reduce forming difficulty of the first anvil unit 11. The anvil 10 has a staple forming surface disposed opposite to the staple cartridge 20, wherein, the anvil forming surface 111 corresponding to the first anvil unit 11 and the anvil forming surface 121 corresponding to the second anvil unit 12 are in wave shape. The surfaces of the staple cartridge 20 and the anvil 10 which are disposed face to face are overall presented in wavy arrangement which is regular and uniform. Besides, referring to Fig. 2, the staple forming surface is configured to be meshed with the working surface of the staple cartridge 20 to minimize the tissues squeezed out and optimize the clamping effect on the target tissues.

The above-mentioned embodiments according to present invention can achieve the beneficial effects as follows: the linear suturing and cutting device provided by the present invention comprises such structure that the cartridge holes in staple cartridge is configured to be inclined towards the distal end of said staple cartridge, then, the direction of force received by the staple pushers is identical with the extending direction of cartridge holes during the staple pushers being pushed in the cartridge holes, thus the staple pushers will not get too large friction from the inner wall of cartridge holes and then can be pushed more smoothly, which reduces the difficulty of operation.

It should be understood that although the description is presented in accordance with these embodiments, but not every embodiment contains only a single technical solution, only for the sake of clarity. Those skilled in the art should take the description as a whole to combine technical solutions in these embodiments to form the other embodiments which can be understood.

A series of detailed instructions listed above are merely a specific description for the practical embodiments of present invention, which are not intended to limit the scope of the present invention. All the equivalent embodiments or variants without departing away from the spirit of present invention should be included within the scope of the present invention.

## Claims

1. A linear suturing and cutting device, comprising an upper jaw and a lower jaw capable of being closed or opened relative to each other;
said upper jaw includes an anvil;
said lower jaw includes a staple cartridge frame, said staple cartridge frame is detachably provided with a staple cartridge, said staple cartridge is arranged with cartridge holes inside which run throughout said staple cartridge to its working surface and staple pushers correspondingly disposed inside said cartridge holes and being capable of sliding relative to said cartridge holes, said staple cartridge frame is slidably provided with a firing piece inside, said firing piece is configured with a wedge-shape staple pushing member capable of driving said staple pushers to slide in said cartridge holes, wherein, said cartridge holes are configured to be inclined towards distal end of said staple cartridge.

2. The linear suturing and cutting device according to claim 1, wherein, said firing piece moves from the proximal end to the distal end of said staple cartridge, said wedge-shape staple pushing member has a staple pushing surface engaged with said staple pushers, said staple pushing surface is configured to be sloped upward in the direction from the distal end towards the proximal end.

3. The linear suturing and cutting device according to claim 2, wherein, said cartridge holes are vertically arranged relative to said staple pushing surface.

4. The linear suturing and cutting device according to claim 1, wherein, upper end surface of each said cartridge hole is an upward inclined plane in the extending direction from the distal end towards the proximal end of the staple cartridge.

5. The linear suturing and cutting device according to claim 4, wherein, said anvil includes a first anvil unit and at least one second anvil unit which is fixed at either side of said first anvil unit, and said first anvil unit and said second anvil unit are manufactured separately.

6. The linear suturing and cutting device according to claim 5, wherein, said staple cartridge includes a cutter groove arranged along the major longitudinal axis thereof, the pattern of said cartridge holes by either side of said cutter groove presents as that each two adjacent rows of cartridge holes are in a staggered arrangement.

7. The linear suturing and cutting device according to claim 6, wherein, the patterns of said cartridge holes by two side of said cutter groove are symmetrical with each other.

8. The linear suturing and cutting device according to claim 6, wherein, said anvil has a staple forming surface disposed opposite to the staple cartridge, wherein, part of said staple forming surface corresponding to said first anvil unit and part of said staple forming surface corresponding to said second anvil unit are in wave shape.

9. The linear suturing and cutting device according to claim 8, wherein, said staple forming surface is configured for engaging with said working surface of said staple cartridge.

10. A linear suturing and cutting device, comprising an upper jaw and a lower jaw capable of being closed or opened relative to each other, said upper jaw includes an anvil, said lower jaw includes a staple cartridge frame, said staple cartridge frame is detachably provided with a staple cartridge, said staple cartridge is arranged with cartridge holes inside which run throughout said staple cartridge to its working surface, and staple pushers correspondingly arranged inside said cartridge holes and being capable of sliding relative to said cartridge holes, said staple cartridge frame is slidably provided with a firing piece inside, said firing piece is configured with a wedge-shape staple pushing member capable of driving said staple pushers to slide in said cartridge holes, wherein, said cartridge holes are configured such that the direction of pushing force received by said staple pushers, while said wedge-shape staple pushing member drives said staple pushers to slide in said cartridge holes, is same with the extending direction of said cartridge holes.

11. The linear suturing and cutting device according to claim 10, wherein, said cartridge holes are configured to be inclined towards the distal end or proximal end of said staple cartridge.

12. The linear suturing and cutting device according to claim 11, wherein, said wedge-shape staple pushing member has a staple pushing surface engaged with said staple pushers, said cartridge holes are perpendicular to said staple pushing surface.
